# EUROPEAN PATENT APPLICATION

(11) **EP 4 261 285 A1**
(43) Date of publication of application: **18.10.2023**
(21) Application number: 21903051.7
(22) Date of filing: 28.10.2021
(51) Int. Cl.: C12N 15/115, A61K 31/7088, A61K 47/54, A61P 7/02, C07K 14/745

(54) **NEUTRALIZABLE COVALENT DRUG**

(30) Priority: 08.12.2020 JP 2020203420
(71) Applicant: Tabuchi, Yudai, Chofu-shi, Tokyo 182-8585 (JP); Yang, Jay, Sapporo-shi, Hokkaido 064-0821 (JP); Taki, Masumi, Chofu-shi, Tokyo 182-8585 (JP)
(72) Inventor: Tabuchi, Yudai, Chofu-shi, Tokyo 182-8585 (JP); Yang, Jay, Sapporo-shi, Hokkaido 064-0821 (JP); Taki, Masumi, Chofu-shi, Tokyo 182-8585 (JP)
(74) Representative: Grünecker Patent- und Rechtsanwälte PartG mbB
(86) International application number: PCT/JP2021/039891
(87) International publication number: WO 2022/123947

(57) **Abstract**

Provided is a novel drug modality which at least partially overcomes the shortcomings of the conventional covalent drugs. A neutralizable covalent drug compound comprising a nucleic acid aptamer and a fluorosulfonyl group linked to the nucleic acid aptamer via a linker is provided. A composition comprising the compound and a method of producing the compound are also provided. Also provided is a neutralizable covalent drug system, comprising the compound or the composition and an oligonucleotide complementary to the nucleic acid aptamer.

## Description

### Technical Field

The present disclosure relates to compounds and compositions which specifically bind to targets and which are useful as medicaments or as biochemical tools.

### Background Art

Development of covalent drugs (covalent-binding agents), which form covalent bonds specifically to the target proteins, has been put in the spotlight in recent years (Non-Patent Document 1). Based on the formation of the covalent bond, a covalent drug can exert its drug effect semi-permanently. Since the covalent drugs can, compared to ordinary (*i.e*., non-covalent-binding) drugs, exert their effects at lower dosing frequencies and/or lower doses, they are attracting interest as next-generation drug candidates which may improve quality of life of the patients.

On the other hand, because of their characteristics the covalent drugs have a potential to cause side effects or toxicity which may be long-lasting and difficult to remove or antidote, and therefore their safety is a concern. Although covalent-binding modalities based on a variety of molecules including small molecules and peptides have been developed so far (Non-Patent Documents 2 and 3), no covalent-binding modality has emerged as being amenable to efficient neutralization, antidoting, and/or drug effect control.

Non-Patent Document 4 reports introducing a sulfonyl fluoride group at the 5'-ends of a SELEX (Systematic Evolution of Ligands by Exponential enrichment) library to perform a SELEX screening, from which a DNA aptamer was obtained having an ability to bind the epidermal growth factor receptor (EGFR). However, although Non-Patent Document 4 has confirmed the aptamer's intrinsic ability to bind the target (*i.e*., its ability to achieve docking to the target EGFR), the ability to form a covalent bond to the EGFR does not appear to have been verified adequately.

### Prior Art Literature

### Non-Patent Documents

Non-Patent Document 1: Singh, J.; Petter, R. C.; Baillie, T. A.; Whitty, A., The resurgence of covalent drugs. Nat Rev Drug Discov 2011, 10 (4), 307-17.
Non-Patent Document 2: Bauer, R. A., Covalent inhibitors in drug discovery: from accidental discoveries to avoided liabilities and designed therapies. Drug Discov Today 2015, 20 (9), 1061-73.
Non-Patent Document 3: Gambini, L.; Baggio, C.; Udompholkul, P.; Jossart, J.; Salem, A. F.; Perry, J. J. P.; Pellecchia, M., Covalent Inhibitors of Protein-Protein Interactions Targeting Lysine, Tyrosine, or Histidine Residues. Journal of Medicinal Chemistry 2019, 62 (11), 5616-5627.
Non-Patent Document 4: Yuko SHISHIDO, Hokkaido University Doctoral Thesis, September 25, 2018

### Summary of the Invention

### Problem to Be Solved by the Invention

The present disclosure provides a novel drug modality which at least partially overcomes the shortcomings of the conventional covalent drugs. In another aspect the present disclosure provides an aptamer-based drug which can have an altered pharmacokinetics.

### Solution to the Problem

The present disclosure includes the following embodiments.
[1] A neutralizable covalent drug compound, comprising a nucleic acid aptamer and a fluorosulfonyl group linked to the nucleic acid aptamer via an azide-alkyne click chemistry reaction,
   wherein the aptamer and the fluorosulfonyl group are linked via a linker comprising a linking moiety formed by the azide-alkyne click chemistry reaction.
[2] The neutralizable covalent drug compound according to [1],
   wherein the linker is represented by a formula -L¹-Y-L²-, wherein Y is the linking moiety formed by the azide-alkyne click chemistry reaction, L¹ is a first linker moiety bound to the nucleic acid aptamer, and L² is a second linker moiety bound to the fluorosulfonyl group,
   wherein within the second linker moiety, the end portion bound to the fluorosulfonyl group is an arylene.
[3] The neutralizable covalent drug compound according to [2], wherein the arylene of the end portion bound to the fluorosulfonyl group is provided in the following structure: 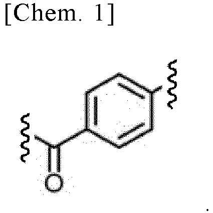
[4] The neutralizable covalent drug compound according to any one of [1]-[3], wherein the linker is bound to a nucleobase in the nucleic acid aptamer.
[5] The neutralizable covalent drug compound according to any one of [1]-[4], wherein the nucleic acid aptamer is a thrombin-binding aptamer having the sequence: 5'-GGTTGGTGTGGTTGG-3' (SEQ ID NO:1).
[6] A pharmaceutical composition comprising the neutralizable covalent drug compound according to any one of [1]-[5].
[7] An anticoagulant agent comprising the neutralizable covalent drug compound according to [5].
[8] The anticoagulant agent according to [7] for use in a method of preventing or inhibiting blood coagulation in a patient, wherein the method comprises administering the anticoagulant agent to the patient and, following the administration of the anticoagulant agent, further administering an oligonucleotide complementary to the nucleic acid aptamer.
[9] A neutralizable covalent drug system, comprising:
   the compound according to any one of [1]-[5], the composition according to [6], or the anticoagulant agent according to [7] or [8]; and
   an oligonucleotide complementary to the nucleic acid aptamer.
[10] A method of producing a neutralizable covalent drug capable of forming a covalent bond to a target protein, the method comprising reacting:
   a) a nucleic acid aptamer specific to the target protein, wherein an alkynyl, cycloalkynyl, or heterocycloalkynyl group (a1) or an azide group (a2) is linked or bound to the aptamer; and
   b) a warhead compound having a structure in which a corresponding azide group (b1) or alkynyl, cycloalkynyl, or heterocycloalkynyl group (b2) is linked or bound to a fluorosulfonyl group

   to carry out an azide-alkyne click chemistry reaction, and
   obtaining a structure in which the nucleic acid aptamer and the fluorosulfonyl group are linked via a linker comprising a linking moiety formed by the azide-alkyne click chemistry reaction.
[11] The method according to [10], wherein the warhead compound comprises a second linker linking the fluorosulfonyl group to the azide group or the alkynyl, cycloalkynyl, or heterocycloalkynyl group, wherein within the second linker, the end portion bound to the fluorosulfonyl group is an arylene.
[12] The method according to [11], wherein the arylene of the end portion bound to the fluorosulfonyl group is provided in the following structure: 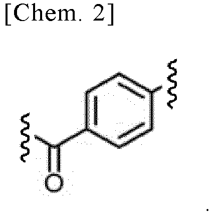
[13] A neutralizable covalent drug compound, comprising a nucleic acid aptamer; and a fluorosulfonyl group linked to the nucleic acid aptamer via a linker, wherein within the linker, the end portion bound to the fluorosulfonyl group is an arylene.
[14] The neutralizable covalent drug compound according to [13], wherein the arylene of the end portion bound to the fluorosulfonyl group is provided in the following structure:

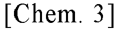

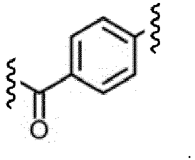

### Effects of the Invention

The compounds and compositions according to the embodiments of the present disclosure not only have the properties of covalent drugs, but they can also allow controlling of drug effect at a desired timing to prevent, remove, or antidote potential side effects or toxicity. They can therefore have utility as covalent drugs embodying a novel drug modality. This modality improves safety of covalent drugs and thus may accelerate applications of covalent drugs in medicine.

### Brief Descriptions of the Figures

[Figure 1] Figure 1 provides HPLC data showing efficiency of introducing the fluorosulfonyl group into the thrombin-binding aptamers (TBAs) having the linker attached to different positions.
[Figure 2] Figure 2 provides data from the mobility shift experiments showing whether or not the TBAs linked to the fluorosulfonyl group form a covalent bond to the target.
[Figure 3] Figure 3 (a) shows a comparison of CD spectra of the unmodified TBA, alkyne-linker-modified TBA, and warhead-modified TBA. (b) provides data showing that the warhead-modified TBA competes with the unmodified TBA for binding to thrombin.
[Figure 4] Figure 4 shows results of the experiments investigating concentration dependency in the covalent bonding reaction by the warhead-modified TBA.
[Figure 5] Figure 5 provides data showing that the warhead-modified TBA specifically binds to and forms a covalent bond to thrombin in the presence of serum proteins, and further its complementary strand specifically binds to the covalently bound complex. The complementary strand is labeled with FAM and detected by fluorescence imaging.
[Figure 6] Figure 6 shows the results of investigating inhibition of thrombin activity by TBA, by looking at the ability to produce polymerized fibrin from fibrinogen. (a) shows accumulation rates of polymerized fibrin in the presence or absence of the unmodified TBA or the warhead-modified TBA₃. (b) and (c) show concentration-dependency of the unmodified TBA (b) and the warhead-modified TBA₃ (c) for the inhibition of thrombin activity.
[Figure 7] Figure 7 shows formation of covalent bonds to the targets by the SARS-CoV-2 spike protein binding aptamer (a, b) and the VEGF binding aptamer (c) having a fluorosulfonyl group linked at various positions.
[Figure 8] Figure 8 (a) and (b) provide data showing neutralization of the warhead-modified aptamer by the complementary strand.

### Detailed Description of the Invention

The present disclosure in one aspect provides a compound comprising a nucleic acid aptamer and a fluorosulfonyl group linked to the nucleic acid aptamer via a linker. More specifically, in one embodiment, a compound comprising a nucleic acid aptamer and a fluorosulfonyl group linked to the nucleic acid aptamer via an azide-alkyne click chemistry reaction is provided. Alternatively, or in addition, provided in some embodiments is a compound comprising a nucleic acid aptamer and a fluorosulfonyl group linked to the nucleic acid aptamer via a linker, wherein the end portion within the linker bound to the fluorosulfonyl group is an arylene. In the present disclosure, these compounds may be also referred to as neutralizable covalent drug compounds. By having such a structure, the compound can specifically recognize and bind the target via the nucleic acid aptamer moiety, and forms a covalent bond to the target via the fluorosulfonyl group.

In the context of the present embodiments, "drug" refers to a substance that can specifically bind to a target substance, particularly a target protein. Thus, for example, a drug that can bind to the active site of a target enzyme to inhibit the enzymatic activity is especially preferable, but a drug does not necessarily have to have such an effect, and those drugs which are used simply as binding labels or targeting moieties, for example, are also contemplated. Therefore, the term "drug" should be construed as referring to a "binding agent" that can be broadly used *in vivo* or *in vitro.* In one aspect, the compound, or covalent drug compound, of the present embodiments can be understood as a binding agent which specifically recognizes and binds the target via the aptamer moiety. The target which is specifically recognized and bound may be a single specific substance, or specific substances having common or similar structures. A "covalent drug" means it is a drug further having an ability to form a covalent bond to the target.

The nucleic acid aptamer technology *per se* is well known to those skilled in the art. Naturally occurring nucleic acid aptamers existing as part of riboswitches are also known, but more typical nucleic acid aptamers in the technological applications are artificial nucleic acid aptamers, and more specifically, nucleic acids selected by screening random sequence libraries for the ability to bind the target substances, or nucleic acids obtained by partially modifying (e.g., truncating and/or chemically modifying) the nucleic acid sequences thus selected. These are usually non-naturally occurring free nucleic acid fragments comprising (or consisting of) sequences that are not naturally present, or at least, non-naturally occurring free nucleic acid fragments comprising (or consisting of) sequences that do not encounter the target molecules in a natural condition. Since an aptamer is obtained or produced as an entity which binds to a target substance of interest, the specific target is accordingly known for the respective aptamer. Therefore, a person skilled in the art can clearly recognize a nucleic acid aptamer and its corresponding target.

Many aptamers have been published and may be employed in the present embodiments. Examples include, but are not limited to, the aptamers binding to thrombin, coagulation Factor IXa, factor Xa, von Willebrand factor, tissue factor pathway inhibitor, vascular endothelial growth factor, platelet-derived growth factor, complement protein C5, nucleolin, CXCL12, CCL2, hepcidin, Toll-like receptor 9, SARS-CoV-2 spike protein, VEGF and the like, which are described in the publications mentioned in the present application or those cited by these publications. Further examples of aptamers and descriptions of known technologies and reagents relating to the aptamers can be found in e.g., aptagen.com, bioanalysis-zone.com, genemedsyn.com, linaris.de, cambio.co.uk, and basepairbio.com.

A nucleic acid aptamer can specifically bind to the target substance with a sub-micromolar (preferably sub 100 nanomolar, more preferably sub-10 nanomolar) binding affinity as expressed by dissociation constant K_{d}, and the nucleic acid aptamers of the present embodiments linked to the fluorosulfonyl groups may also retain such specific binding ability and affinity for the target substances. Typically, binding between the aptamer and the target is obtained as a sum total of numerous non-covalent binding interactions.

A nucleic acid aptamer may be a DNA, an RNA, or a combination thereof, and may be, for example, a single-stranded DNA, a single-stranded RNA, or a combination thereof. Some single-stranded nucleic acid aptamers may partially form self-complementary strands within the molecules. The term nucleic acid aptamer encompasses those comprising one or more nucleic acid modifications or non-natural nucleic acid moieties (such as non-natural backbones and non-natural sugar moieties) known to a person skilled in the art. Various nucleic acid modifications and non-natural nucleic acid moieties which can be used within the aptamers, *e.g*. for the purpose of nuclease resistance or the like, are known (Zhou et al., Nat Rev Drug Discov, 2017, 16(3): 181-202). Examples thereof include, but are not limited to, 2'-fluorination, 2'-amination, 2'-O-methylation, 3'-modification by an inverted thymidine, phosphorothioate, LNA, and polyethylene glycol (PEG) modifications. The length of the nucleic acid aptamer is typically 10 nucleotides (bases) or longer, more typically 15 nucleotides or longer. The length of the nucleic acid aptamer is typically no longer than 100 nucleotides and more typically no longer than 50 nucleotides, but it can be longer than these.

The term "neutralization" as used in the context of the present embodiments means canceling of the binding (or binding ability) of the aptamer moiety of the present drug or compound, and "neutralizable" means such neutralization can be artificially induced. With the cancelation of the binding of the aptamer moiety, it is understood that the pharmacological effect mediated by the binding of the aptamer moiety itself can also be canceled. For example, if the drug has an aptamer moiety which binds to an active site of the target enzyme to prevent the access of the substrate and thereby inhibit the enzyme, neutralization can cancel the enzyme-inhibiting effect. Neutralization may be partial neutralization, in which case lessening of the drug effect, rather than complete loss of the drug effect, may be achieved. Alternatively, if the drug has an aptamer moiety, which is causing a side effect through binding, neutralization can cancel the side effect.

More specifically, neutralization can be effected by adding a complementary strand to the nucleic acid aptamer to interfere with the structure of the aptamer-target interaction. Such a complementary strand may be referred to as an antidote, but this does not mean the drug or compound is necessarily poisonous - an antidote should be plainly understood as an agent for effecting neutralization as defined above. The antidotes will be described in more detail below. Moreover, "neutralization" merely means cancelation of the binding (or binding ability) of the aptamer moiety itself, and the covalent bond newly formed via the fluorosulfonyl group is not canceled (or severed) by the "neutralization". In fact, it is believed that neutralization by adding an antidote will result in a condition in which the nucleic acid aptamer moiety, whose binding to the target has been canceled, is tethered to the target molecule by the linker stemming from the covalent binding point which was formed via the fluorosulfonyl group.

The fluorosulfonyl group may be represented by the formula -SO₂F and is a reactive, electrophilic chemical group known to be capable of reacting with at least a serine, threonine, lysine, tyrosine, cysteine, or histidine residue of a protein under physiological conditions to form a covalent bond (Narayanan et al., Chemical Science 2015, 6(5), 2650-2659). Such a reactive group forming a covalent bond with a target may be referred to as a warhead. In the present disclosure, a nucleic acid aptamer linked to a fluorosulfonyl group may be referred to as "warhead-modified aptamer". As partly illustrated by Narayanan *et al.* paper, diverse compounds having fluorosulfonyl groups have been synthesized, and diverse synthetic routes are available to a person skilled in the art for introducing a fluorosulfonyl group.

A person skilled in the art will appreciate that a wide variety of chemical linkers can be used in the present embodiments as long as they can covalently link the nucleic acid aptamer and the fluorosulfonyl group. Chemical linkers typically have hydrocarbon-based backbones. Hydrocarbon-based backbones include those comprising other atoms bound to and/or inserted into the hydrocarbon backbones. The term "linked" as used in the present disclosure means a condition in which two moieties are indirectly bound by having other atom(s) in between, which is distinguished from a condition in which two moieties are directly covalently bound to each other without having other atoms in between. That is, if one moiety (nucleic acid aptamer) is bound to one end of the linker structure, and another moiety (fluorosulfonyl group) is bound to the other end of the linker structure, then the two moieties are "linked". "Covalently linked" means that the nucleic acid aptamer and the fluorosulfonyl group are connected by a series of covalent bonds, *i.e*., one end of the linker is covalently bound to the nucleic acid aptamer and the other end to the fluorosulfonyl group and the backbone of the linker itself consists of covalent bonds between atoms.

A person skilled in the art can select and use suitable linkers based on ordinary knowledge and depending on the parameters of the linkers being designed such as its lengths, hydrophilicity, flexibility, and steric hindrance as well as convenience of synthesis. For example, the linker may be, but is not limited to, a divalent group comprising or consisting of a C₁₋₁₀ alkylene, C₂₋₁₀ alkenylene, C₂₋₁₀ alkynylene, heteroalkylene, cycloalkylene, heterocycloalkylene, arylene, heteroarylene or other divalent heterocyclic group, -CONH- (or -NHCO-), -CO-, -NH-, -O-, or a combination thereof. Heteroalkylene refers to an alkylene skeleton in which the carbon atom is replaced by one hetero atom per site such as an oxygen, nitrogen, or sulfur atom, and includes ether moieties as well as polyether moieties such as polyethyleneoxy and polypropyleneoxy. A linker not comprising -S- moiety is also contemplated. Cycloalkylene and heterocycloalkylene refer to alkylene and heteroalkylene that are cyclic, respectively. Heterocycloalkylene includes crown ethers. The groups that constitute linkers in the present disclosure include substituted and unsubstituted ones. For example, the hydrogen atoms of alkylene, arylene, heteroarylene, heteroring, and/or -NH- may be substituted. Examples of typical substitution groups include, but are not limited to, C₁₋₁₀ alkyl and alkyloxy, aryl, hydroxyl, amino, halogen, and combinations thereof.

The linker in the embodiments in which the fluorosulfonyl group is linked to the nucleic acid aptamer via an azide-alkyne click chemistry reaction comprises a linking moiety formed by the azide-alkyne click chemistry reaction. Linking moieties formed by other reactive groups and reaction mechanisms may also be feasible in some cases, but by using an azide-alkyne click chemistry reaction, linking of the nucleic acid aptamer and the fluorosulfonyl group and production of the neutralizable covalent drug compound can be performed extremely efficiently.

However, the significance of linking the fluorosulfonyl group to the nucleic acid aptamer via an azide-alkyne click chemistry reaction does not stop at mere efficiency of reaction. Non-Patent Document 4 disclosed attaching a linker having a terminal thiol functional group (-SH) to the 5'-ends of a SELEX library and reacting this with a warhead compound having the structure of vinyl-SO₂F to provide a library in which the warhead -C₂H₄-SO₂F (also called ESF) was linked via a thioether, and then performing a screening therefrom to obtain a target-binding aptamer of interest. However, the present inventors have found that the warhead-modified aptamer using such a linking method lacked the ability to form a covalent bond (see Example section of the present application). Indeed, Non-Patent Document 4 appears to have not confirmed covalent bond formation between the aptamer and the target protein under a fully reducing and denaturing condition as in Examples of the present application. The presence of binding (docking) ability of the aptamer moiety notwithstanding, it is possible that absence of covalent-bonding ability was overlooked. On the other hand, when the present inventors linked the same ESF warhead to the aptamer via an azide-alkyne click chemistry reaction, a clear covalent-bonding ability for the target was acquired. Thus, this approach of linking a warhead via an azide-alkyne click chemistry reaction is a feature having such a significance that one may even say a previously incomplete invention could finally (and surprisingly) see completion through the use of this approach. A possible reason for such a difference in covalent-bonding ability occurring depending on the linking methods could be that the reaction condition for azide-alkyne click chemistry, which can be carried out in a mild solution condition in a short time, can suppress instability or degradation of the warhead compound. Other possibilities could also be considered, for example the liking structure comprising the nitrogen-rich ring provides some kind of favorable physicochemical effect on the nucleic acid, target protein, and/or -SO₂F group.

Azide-alkyne click chemistry reactions *per se* are known, and a person skilled in the art can clearly recognize the structure of the linking moiety formed by an azide-alkyne click chemistry reaction. An azide-alkyne click chemistry reaction can be described as a [3+2] cycloaddition reaction occurring between an azide group (-N=N⁺=N⁻) and a carbon-carbon triple bond (-C≡C-) moiety. One preferable example of the linking moiety formed by an azide-alkyne click chemistry reaction is the divalent group represented by the chemical formula (I) below:

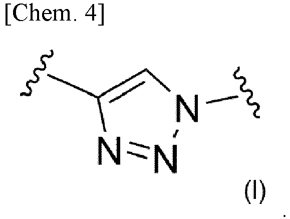

This is a linking moiety formed by the azide-alkyne click chemistry reaction called copper(I)-catalyzed azide-alkyne cycloaddition (CuAAC). In a preferable embodiment, the left side (left arm) of the above structural formula is linked or bound to the nucleic acid aptamer and the right side (right arm) is linked or bound to the fluorosulfonyl group, but the reverse is also possible.

Another example of a linking moiety formed by an azide-alkyne click chemistry reaction is the divalent group represented by general formula (II) below. In general formula (II) below, the 8-membered ring may be substituted by substitution groups (e.g. methyl, methoxy, O=, fluorine atom, or fusion of an aromatic ring or aliphatic ring), and may be for example part of a bicyclo[6.1.0] structure. Further, in general formula (II) below, the 8-membered ring may be a hetero ring in which one or more carbons not taking part in fusion with another ring are replaced by a non-carbon atom such as nitrogen; Typically, the carbon at the site of attachment to the linker (the left bond arm in the general formula (II) below) may be replaced by nitrogen.

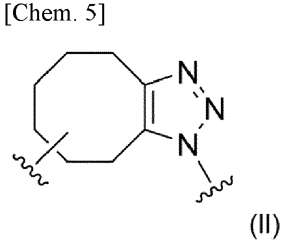

A specific example of the linking moiety represented by the general formula (II) is provided in the chemical formula (IIa) below.

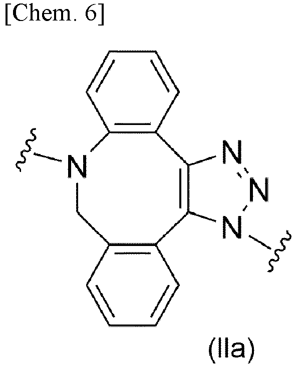

Another specific example of the linking moiety represented by the general formula (II) is provided in the chemical formula (IIb) below.

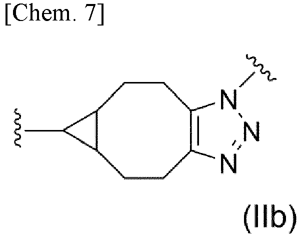

The structures represented by formulas (II), (IIa) and (IIb) are examples of linking moieties formed by azide-alkyne click chemistry reactions called strain-promoted azide-alkyne cycloaddition (SPAAC). SPAAC does not require use of a copper catalyst and therefore is also called a copper-free click chemistry reaction.

The left sides (left arms) of the above structural formulas may be linked or bound to the nucleic acid aptamers and the right sides (right arms) to the fluorosulfonyl groups, or *vice versa.* As described below, the direction of the linking moiety is reversed depending on whether the azide group is provided from the nucleic acid aptamer side or from the fluorosulfonyl group side.

The remaining portions of the linker may be combinations of the groups described above. For example, within the linker, the nucleic acid aptamer side and/or the fluorosulfonyl group side relative to the linking moiety formed by the azide-alkyne click chemistry reaction may be a divalent group comprising or consisting of a C₁₋₁₀ alkylene, C₂₋₁₀ alkenylene, C₂₋₁₀ alkynylene, heteroalkylene, cycloalkylene, heterocycloalkylene, arylene, heteroarylene, -CONH- (or -NHCO-), -CO-, -NH-, -O-, or a combination thereof.

The linker linking the nucleic acid aptamer and the fluorosulfonyl group via an azide-alkyne click chemistry reaction may be represented by formula -L¹-Y-L²-, wherein Y is a linking moiety formed by the azide-alkyne click chemistry reaction, L¹ is a linker moiety bound to the nucleic acid aptamer (corresponding to the first linker described below), and L² is a linker moiety bound to the fluorosulfonyl group (corresponding to the second linker described below). -L¹-Y-L²- as a whole corresponds to the third linker described below. In one specific example, the third linker consists of the structure -C≡C-(CH₂)₄-(I)-CH₂-CO-C₆H₄-, wherein (I) represents the chemical formula (I).

Within L², the end portion bound to the fluorosulfonyl group is preferably an arylene (*e.g.*, *p*-phenylene) or heterocycloalkylene (*e.g.*, 1,4-piperazinylene). In such embodiments, the end portion bound to the fluorosulfonyl group especially preferably has the following structure, *i.e.* carbonyl phenylene, as a structure providing said arylene.

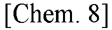

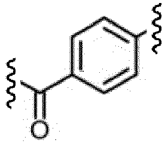

For example, L² (the second linker moiety) may be an acetyl-benzene moiety, *i.e.* methylene-carbonyl-phenylene.

Determining how different warhead structures might behave in the context of aptamer-based covalent drugs was an unexplored territory and there was a high level of unpredictability. Here, it has been found that the aryl-type warheads in which the fluorosulfonyl group is provided as an arylene-bound structure as above can provide, in the context of aptamer-based covalent drugs, substantially higher ability to form a covalent bond to the aptamer target, *e.g.* compared to the alkyl-type warhead used in Non-Patent Document 4. The difference in the covalent-bonding ability is significant; The aryl-type warhead could provide a covalent-bonding ability against the aptamer target even when linked by using linking methods other than the azide-alkyne click chemistry reactions, such as the thiol-mediated method as used in Non-Patent Document 4.

Accordingly, the present disclosure in one aspect provides a neutralizable covalent drug compound comprising a nucleic acid aptamer and a fluorosulfonyl group linked to the nucleic acid aptamer via a linker, wherein within the linker, the end portion bound to the fluorosulfonyl group is an arylene. Arylene is especially preferably provided in the form of, *e.g*., the above-described carbonyl-phenylene or acetyl-benzene moiety. In this embodiment, the linkage between the nucleic acid aptamer and the fluorosulfonyl group may not be via azide-alkyne click chemistry, and a linkage or a linking moiety formed by other reactive groups and reaction mechanisms will be feasible. However, linking the aryl-type warhead to the nucleic acid aptamer via azide-alkyne click chemistry is preferable because it may provide a significant synergistic effect in covalent bonding efficiency against the aptamer target.

As described above, the length and specific composition of the linker may be varied depending on the practitioner's design, but just as a guide, the number of carbons in the linker may typically be within the range of 2-100, more typically 5-50. Also, just as a guide, the length of the linker, *i.e*., the distance between the nucleic acid aptamer and the fluorosulfonyl group when the linker is maximally extended may typically be no longer than 100Å, more typically no longer than 50Å, or no longer than 30Å. The length of the linker is typically 5Å or longer, more typically 10Å or longer. When the linker is made longer, covalent bonding by the fluorosulfonyl group becomes possible at a site farther from the aptamer-bound site, or at a site farther from the linker-attachment point of the aptamer. The covalent bond is usually formed within the target molecule, but there is a possibility that a covalent bond is formed to other molecules which are present near the target molecule during the aptamer binding.

It has been discovered that within the nucleic acid aptamer, the location to which the linker is attached may be varied relatively freely. A person skilled in the art can determine a suitable location for linker attachment in the nucleic acid aptamer as needed and without undue experimentation. In a preferable embodiment, the linker is bound to a nucleobase in the nucleic acid aptamer. The linker may be bound to a non-5' and non-3' end, e.g., to an internal nucleobase. In another embodiment, the linker may be bound to a 5'-end or a 5'-terminal phosphate (*i.e*., a phosphate group bound to the 5'-end) of the nucleic acid aptamer. In another embodiment, the linker may be bound to a 3'-end or a 3'-terminal phosphate (*i.e*., a phosphate group bound to the 3'-end) of the nucleic acid aptamer, or to the 3'-position of the ribose ring of any of the nucleotides/nucleosides (in which case 2'-5'-phosphodiester linkage may be utilized). In still other embodiments, the linker may be bound to the 2'-position of the ribose ring of any of the nucleotides/nucleosides constituting the nucleic acid aptamer.

Nucleotides/nucleosides having alkyne structures or azide groups bound or linked to these locations, which are usable for azide-alkyne click chemistry reactions, as well as oligonucleotides of any sequences incorporating such nucleotides/nucleosides, may be commercially available or synthesized by a person skilled in the art. Oligonucleotide synthesis services for any sequences, incorporating nucleotides having alkyne structures or azide groups bound or linked to any of the above-described locations, are also commercially available. Examples of the companies providing reagents or custom synthesis service for such alkyne-modified or azide-modified oligonucleotides include, but are not limited to, Integrated DNA Technologies, Inc., Nihon Gene Research Laboratories Inc., and Sigma-Aldrich Co. LLC. These structures have been conventionally used for *e.g.* fluorescent labeling of nucleic acids.

When a linker is bound to a nucleobase in the nucleic acid aptamer, the base may be any of adenine (A), guanine (G), inosine (I) (hypoxanthine), cytosine (C), thymine (T), and uracil (U), but A, C, T or U is preferable and T or U is especially preferable. For C and U, for example, the linker may be bound to 5'-position of the pyrimidine ring constituting the base. For T, the linker may be attached by replacing the methyl group at 5'-position of the pyrimidine ring constituting the thymine base, resulting in the same structure as 'U' bound to the linker as described above. In the present specification, such a situation may still be described by saying the linker is bound to 5'-position of the pyrimidine ring constituting the T (thymine) base. For A, G, and I, the linker may be bound to 7'-position of the purine ring constituting the base. In such cases, the nitrogen atom at 7'-position of the purine ring may be replaced by a carbon atom.

In an embodiment, the nucleic acid aptamer constituting part of the subject compound is a thrombin-binding aptamer having the following sequence (SEQ ID NO:1): 5'-GGTTGGTGTGGTTGG-3'. Thus, provided is a compound, or a neutralizable covalent drug compound, comprising a nucleic acid aptamer having the sequence of SEQ ID NO:1 and a fluorosulfonyl group linked to the nucleic acid aptamer via a linker. Thrombin is an enzyme involved in blood coagulation by cleaving fibrinogen to produce fibrin. The thrombin-binding nucleic acid aptamer *per se* is described in Bock et al., Nature, 1992, 355, 564-566. Further nucleic acid sequences may be added to 5'- and/or 3'-side of the above sequence. In fact, Bock *et al.* isolated a plurality of aptamer clones having the ability to bind thrombin by screening populations of 96-nucleotide-long nucleic acids (of which 60 nucleotides provided the random sequence and 36 nucleotides provided primer-binding sequences for PCR amplification), which were much longer than the 15-mer. The above 15-mer was a core sequence which was commonly present (or "conserved") in those plural thrombin-binding clones and which maintained the thrombin-binding ability even in isolation. As in this case, it is typically seen in other nucleic acid aptamers that presence of additional sequences is permitted at 5'- and/or 3'-side of the core sequence providing the target-binding ability.

As already described above, in this embodiment also, the linker may be attached to various sites on the thrombin-binding aptamer. For example, a compound is provided in which the linker is bound to the nucleobase of T at position 3, T at position 9, or T at position 12, of the 15-mer. The thrombin-binding aptamer is a DNA aptamer and therefore it originally has T bases. When the linker is attached by replacing the methyl group at position 5 of the pyrimidine ring of T base, such a linker-modified T base will have the same structure as the corresponding linker-modified U base; However, since the original aptamer has the sequence with T bases, it is described herein as "the linker being bound to T base". When the same linker is bound to a U base of an RNA aptamer, it may be described as "the linker being bound to U base" even if the resulting structure may be the same.

In another embodiment, the nucleic acid aptamer constituting part of the subject compound is a SARS-CoV-2 spike protein-binding aptamer having the following sequence (SEQ ID NO:2): 5'-CAGCACCGACCTTGTGCTTTGGGAGTGCTGGTCCAAGGGCGTTAATGG ACA-3'. Thus, provided is a compound, or a neutralizable covalent drug compound, comprising a nucleic acid aptamer having the sequence of SEQ ID NO:2 and a fluorosulfonyl group linked to the nucleic acid aptamer via a linker. This nucleic acid aptamer *per se* is described by Song et al., Anal. Chem., 2020, 92, 9895-9900. SARS-CoV-2 is a novel corona virus causing a pandemic as of year 2020. Its spike protein is a viral protein that mediates initiation of the infection to a human by binding to the ACE2 receptor expressed on human cells, *e.g.* in the respiratory system. The DNA aptamer of SEQ ID NO:2 was identified by Song *et al.* based on the following benchmarks: It binds to the receptor binding domain (RBD) of the spike protein; and it competes with ACE2 for binding to the RBD. Further nucleic acid sequences may be added to 5'- and/or 3'-side of the above sequence. Also contemplated are embodiments of the compound, or a composition comprising the same, for use in a method of inhibiting binding of SARS-CoV-2 to an ACE2 receptor *in vitro* or *in vivo.* Also contemplated are embodiments of a method of inhibiting binding of SARS-CoV-2 to an ACE2 receptor of a subject, the method comprising administering an effective amount of the compound, or a composition comprising the same, to the subject.

As described above, in these embodiments also, the linker may be attached to various sites on the nucleic acid aptamer. For example, a compound is provided in which the linker is bound to the nucleobase of T at position 12, T at position 29, or T at position 42, of the 5 1-mer of SEQ ID NO:2.

In still another embodiment, the nucleic acid aptamer constituting part of the subject compound is a VEGF-binding aptamer having the following sequence (SEQ ID NO:3): 5'-CAATTGGGCCCGTCCGTATGGTGGGT-3'. Thus, provided is a compound, or a neutralizable covalent drug compound, comprising a nucleic acid aptamer having the sequence of SEQ ID NO:3 and a fluorosulfonyl group linked to the nucleic acid aptamer via a linker. This nucleic acid aptamer *per se* is described by Kaur and Yung, PLoS ONE, 2021, 7:e31196. VEGF (vascular endothelial growth factor) is a strongly angiogenic mitogen overexpressed in a wide variety of cancer cells. This nucleic acid aptamer has a very high target-binding affinity, K_{d} of 0.5±0.32 nM, and was produced by Kaur *et al.* by truncating a longer, existing VEGF-binding aptamer. Further nucleic acid sequences may be added to 5'- and/or 3'-side of the above sequence.

In these embodiments also, the linker may be attached to various sites on the nucleic acid aptamer. For example, a compound is provided in which the linker is bound to the nucleobase of T at position 4, T at position 17, or T at position 22, of the 26-mer of SEQ ID NO:3.

In another aspect, a pharmaceutical composition comprising any of the neutralizable covalent drug compound is provided. In other words, the present disclosure provides the neutralizable covalent drug compound for use as a medicine. Also provided is a method comprising administering the neutralizable covalent drug compound to a subject. The subject receiving the administration or treatment in the present disclosure may be an animal, particularly a mammal. The subject may be a human. In the present disclosure, the term "subject" may be used interchangeably with the term "patient".

The pharmaceutical composition may further comprise a pharmaceutically acceptable carrier or excipient. A typical example of such a carrier or excipient is water. A person skilled in the art can select suitable administration routes as needed to deliver an effective amount of the pharmaceutical composition to the site or region where the target protein is present. Examples of administration routes include, but are not limited to, oral, intravenous, intra-arterial, intramuscular, subcutaneous, transdermal, intraperitoneal, intrathecal, transrectal, transvaginal, and by inhalation.

In one specific aspect, provided is an anticoagulant agent comprising a neutralizable covalent drug compound, wherein the compound comprises a nucleic acid aptamer having the sequence of SEQ ID NO: 1 and a fluorosulfonyl group linked to the nucleic acid aptamer via a linker. In other words, the present disclosure provides the neutralizable covalent drug compound for use as an anticoagulant. The anticoagulants mentioned in the present disclosure are agents that prevent or inhibit blood coagulation. The anticoagulants, also, may further comprise pharmaceutically acceptable carriers or excipients. A typical example of such carriers or excipients is water.

The anticoagulants may find utility in any of *in vitro*, *ex vivo*, and *in vivo* settings. In one embodiment, the anticoagulant, or the compound, is provided for use in a method of preventing or inhibiting blood coagulation in a patient. The present disclosure also provides a method of preventing or inhibiting blood coagulation in a patient, comprising administering an effective amount of the anticoagulant or compound to the patient. The anticoagulant may be administered to the patient by, e.g., intravenous, intraarterial, or oral administration. The patient may be, for example, a patient in need of a long-term anticoagulation treatment. The patient may be, for example, but is not limited to, a patient having thrombosis, deep vein thrombosis, vascular occlusive disease, thromboembolism, myocardial infarction, atrial fibrillation, stroke, systemic inflammatory disease (*e.g.*, those associated with COVID-19), or a patient having a risk, or needing prevention, of these diseases. The patient may be a patient receiving prosthetic vessel replacement.

Alternatively, the patient may be a patient receiving a catheter procedure, prosthetic valve replacement, or a coronary artery bypass graft. In these treatments use of heparin as an anticoagulant is common, but the anticoagulant of the present embodiment may be substituted for the heparin. In particular, in rare patients with heparin induced thrombocytopenia and anti-thrombin deficiency, use of heparin may not be feasible and therefore use of the anticoagulant of the present embodiment may be beneficial.

The above-described method may comprise administering the anticoagulant to the patient, and following the administration of the anticoagulant, further administering an oligonucleotide complementary to the nucleic acid aptamer constituting the anticoagulant. In the present disclosure, the oligonucleotide complementary to the nucleic acid aptamer may be an oligonucleotide capable of specifically hybridizing to the nucleic acid aptamer in a physiological saline (0.9% w/v NaCl) at 37°C. By specifically hybridizing to the nucleic acid aptamer, the complementary oligonucleotide can interfere with the aptamer-target interaction structure and cancel the specific binding of the aptamer to the target. In relation to the nucleic acid aptamer-based drugs, the complementary oligonucleotides may be referred to as "antidotes". Cancellation of nucleic acid aptamer-based binding by their complementary oligonucleotides is an established technique (Rusconi et al., Nat Biotechnol, 2004, 22 (11), 1423-8; Stoll et al., Molecules, 2017, 22 (6), 954; Bompiani et al., Curr Pharm Biotechnol, 2012, 13 (10), 1924-34; see also Examples below). Specific sequences of suitable complementary oligonucleotides may be determined based on ordinary knowledge of a person skilled in the art.

Following the administration of the anticoagulant, specific timing of administering the complementary oligonucleotide can be determined as appropriate by the practitioner. For example, the complementary oligonucleotide may be administered after sufficient efficacy of the anticoagulant has been attained, or after an adverse side effect or an excessive effect has begun to occur. The administration route for the complementary oligonucleotide may be the same as, or different from, the administration route for the anticoagulant.

As appreciated by a person skilled in the art, as long as the nucleic acid aptamer can hybridize to the complementary oligonucleotide, the type of the complementary oligonucleotide in the present disclosure is not particularly limited, and it can be the same as or different from that of the nucleic acid aptamer. For example, an antidote to a DNA aptamer may be an oligonucleotide RNA or an oligonucleotide DNA. Complementary oligonucleotides in the present disclosure also encompass those comprising one or more nucleic acid modifications or non-natural nucleic acid moieties known to a person skilled in the art. The length of the complementary oligonucleotide is also not particularly limited and can be determined as appropriate by a person skilled in the art. It may be, for example, 10-50 nucleotide (base) long. The complementary oligonucleotide may be, for example, complementary over at least half the length of the nucleic acid aptamer. In the present disclosure, a complementary oligonucleotide may be referred to as a complementary strand.

As in the examples described above, the neutralizable covalent drug compound having the nucleic acid aptamer moiety and its complementary oligonucleotide may be provided as, or used as, a combination. Therefore, provided in another aspect is a neutralizable covalent drug system, the system comprising the above-described compound, composition, or anticoagulant, and an oligonucleotide complementary to the nucleic acid aptamer in the compound, composition, or anticoagulant. This system may be provided and/or used as a kit. The compound, composition, or anticoagulant and the complementary oligonucleotide are usually in separate formulations which are administered separately, but these formulations may be provided in the same package or in separate packages. The system or kit may further include an instruction for use describing that the complementary oligonucleotide neutralizes the drug effect of the compound, composition, or anticoagulant. The instruction for use may be for example inserted in the package of the compound, composition, or anticoagulant and/or of the complementary oligonucleotide, or printed on the package.

In another aspect, the present disclosure provides a method of producing a neutralizable covalent drug capable of forming a covalent bond to a target protein, the method comprising reacting:
a) a nucleic acid aptamer specific to the target protein, wherein an alkynyl, cycloalkynyl, or heterocycloalkynyl group (a1) or an azide group (a2) is linked or bound to the aptamer; and
b) a warhead compound having a structure in which a corresponding azide group (b 1) or alkynyl, cycloalkynyl, or heterocycloalkynyl group (b2) is linked or bound to a fluorosulfonyl group

to carry out an azide-alkyne click chemistry reaction, and
obtaining a structure in which the nucleic acid aptamer and the fluorosulfonyl group are linked via a linker comprising a linking moiety formed by the azide-alkyne click chemistry reaction.

It should be appreciated that this production method can produce the described neutralizable covalent drug having the linker comprising the linking moiety formed by the azide-alkyne click chemistry reaction. The structural and functional descriptions provided herein for the production method aspect can be applied to the compounds aspect, and *vice versa.* A person skilled in the art can select any combinations of target proteins and nucleic acid aptamers specific thereto from known ones. The method of this embodiment may be described also as a method of converting a nucleic acid aptamer to a covalent drug, or a method of imparting a covalent-binding functionality to a nucleic acid aptamer.

In the present disclosure, a group having a carbon-carbon triple bond (-C≡C-) usable for an azide-alkyne click chemistry reaction, including alkynyl, cycloalkynyl, or heterocycloalkynyl group, may be referred to as an "alkyne structure".

The alkynyl group may be based on a terminal alkyne or an internal alkyne. Cycloalkynyl and heterocycloalkynyl groups usable for azide-alkyne click chemistry reactions are known to a person skilled in the art, usually 7-membered or 8-membered, and may be substituted with substitution groups (e.g., methyl, methoxy, O=, fluorine atom, or fusion to an aromatic or aliphatic ring). The heterocycloalkynyl group is a cycloalkynyl group in which one or more carbons in its ring is replaced by a non-carbon atom such as nitrogen. Typically, a carbon at the site in the ring which is bound to the linker may be replaced by nitrogen. The cycloalkynyl and heterocycloalkynyl groups may be used for SPAAC described above, thus they may undergo a click chemistry reaction in the absence of copper (I) ion. The azide group may be expressed by the formula -N₃.

Examples of the alkyne structures known to those skilled in the art as usable for SPAAC include monovalent groups derived from OCT (cyclooctyne), MOFO (monofluorinated cyclooctyne), DIFO (difluorinated cyclooctyne), DIMAC (dimethoxyazacyclooctyne), DIFBO (difluorobenzocyclooctyne), DIBO (dibenzocyclooctyne), DIBAC (dibenzoazacyclooctyne), BARAC (biarylazacyclooctynone), and BCN (bicyclo[6.1.0]nonyne), respectively.

A specific example of the heterocycloalkynyl group is shown in chemical formula III and a specific example of cycloalkynyl group is shown in chemical formula IV below.

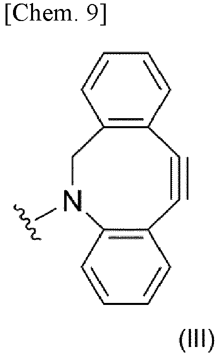

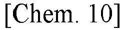

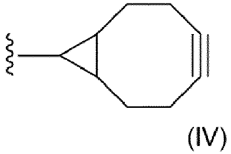

The (a1) or (a2) group may be directly bound to the nucleic acid aptamer, or linked to the nucleic acid aptamer via a linker. In particular, cycloalkynyl groups, heterocycloalkynyl groups, and azide groups are typically linked to the nucleic acid aptamer via a linker. In the present disclosure, the linker linking the nucleic acid aptamer and the (a1) or (a2) group may be called a first linker.

A person skilled in the art can select, and use, a suitable first linker according to the parameters of the linker being designed, such as its length, hydrophilicity, flexibility and steric hindrance, as well as convenience of synthesis. For example the first linker may be, but is not limited to, a divalent group comprising or consisting of C₁₋₁₀ alkylene, C₂₋₁₀ alkenylene, C₂₋₁₀ alkynylene, heteroalkylene, cycloalkylene, heterocycloalkylene, arylene, heteroarylene, -CONH- (or -NHCO-), -CO-, -NH-, -O-, or any combination thereof. The above-listed groups constituting a linker may include substituted and unsubstituted ones. For example, a hydrogen atom of an alkylene, arylene, heteroarylene, and/or -NH- may be substituted. Examples of typical substitution groups include, but are not limited to, C₁₋₁₀ alkyl and alkyloxy, aryl, hydroxyl, amino, halogen, and combinations thereof.

Non-limiting examples of the first linker linking the nucleic acid aptamer (left) to the alkynyl group (right) are recited below: -(CH₂)ₙ- (where n is an integer between 1 and 10); -O-CH₂-; -(CH₂)ₙ-NHCO-(CH₂)₂-O-CH₂- (where n is an integer between 1 and 10); -C≡C-(CH₂)ₙ- (where n is an integer between 1 and 10); and -C≡C-(CH₂CH₂O)ₙ-(CH₂)₂- (where n is an integer between 1 and 10). A specific example of the first linker is -C≡C-(CH₂)₄-.

Non-limiting examples of the first linker linking the nucleic acid aptamer (left) to the cycloalkynyl or heterocycloalkynyl group (right) are recited below: - (CH₂CH₂O)ₙ-(CH₂)₂-NHCO-(CH₂)₄-(CO)- (where n is an integer between 1 and 10); - (CH₂)ₙ-NHCO-(CH₂)₄-(CO)- (where n is an integer between 1 and 10); -(CH₂)ₙ-(where n is an integer between 1 and 10); -(CH₂)₂-O-(CH₂)₂-NHCOO-CH₂-; -(CH₂)ₙ-NHCOO-CH₂- (where n is an integer between 1 and 10); -(CH₂)₆-NHCO-(CH₂)₃-CONH-(CH₂CH₂O)₂-(CH₂)₂-NHCOO-CH₂-.

When the (b) group is said to "correspond" to the (a) group, it means that the (a) group and the (b) group have such a relationship that their combination is capable of undergoing an azide-alkyne click chemistry reaction. Thus, if the (a) group is an alkyne structure group (a1) then the (b) group is an azide group (b1), and conversely, if the (a) group is an azide group (a2) then the (b) group is an alkyne structure group (b2).

The term warhead compound refers to one of the two compounds involved in the linking reactions described in the present disclosure, e.g., azide-alkyne click chemistry reactions, and means the compound having the fluorosulfonyl group which provides the covalent-bonding ability of the covalent drug. The (b 1) or (b2) group of the warhead compound may be directly bound to the fluorosulfonyl group or linked to the fluorosulfonyl group via a linker. In the present disclosure, the linker linking the fluorosulfonyl group and the (b 1) or (b2) group may be called a second linker.

A person skilled in the art can select, and use, a suitable second linker according to the parameters of the linker being designed, such as its length, hydrophilicity, flexibility and steric hindrance, as well as convenience of synthesis. For example the second linker may be, but is not limited to, a divalent group comprising or consisting of C₁₋₁₀ alkylene, C₂₋₁₀ alkenylene, C₂₋₁₀ alkynylene, heteroalkylene, cycloalkylene, heterocycloalkylene, arylene, heteroarylene, -CONH-(or -NHCO-), -CO-, -NH-, -O-, or any combination thereof. The above-listed groups constituting a linker include substituted and unsubstituted ones. For example, a hydrogen atom of an alkylene, arylene, heteroarylene, and/or -NH- may be substituted. Examples of typical substitution groups include, but are not limited to, C₁₋₁₀ alkyl and alkyloxy, aryl, hydroxyl, amino, halogen, and combinations thereof. In particular, the end portion of the second linker bound to the fluorosulfonyl group is preferably an arylene (e.g. p-phenylene) or a heterocycloalkylene (e.g. 1,4-piperazinylene). For example, the end portion of the second linker bound to the fluorosulfonyl group may be an arylene, wherein the arylene may be provided in the form of carbonyl phenylene described above. The second linker may be an acetyl-benzene moiety. These groups constituting the linker may include substituted and unsubstituted ones. For example, a hydrogen atom of an alkylene, arylene, heteroarylene, and/or -NH- may be substituted. Examples of typical substitution groups include, but are not limited to, C₁₋₁₀ alkyl and alkyloxy, aryl, hydroxyl, amino, halogen, and combinations thereof. A specific example of the second linker is -CH₂-CO-C₆H₄-.

Some specific but non-limiting examples of the warhead compounds are shown in chemical formulas (V)-(X) below. In each formula, the moiety linking the azide group and the fluorosulfonyl group is the second linker. The compounds shown below all have an azide group, but the warhead compounds having a (b2) group, *i.e.* an alkynyl, cycloalkynyl, or heterocycloalkynyl group, or a still different reactive group, instead of the azide group are also contemplated.

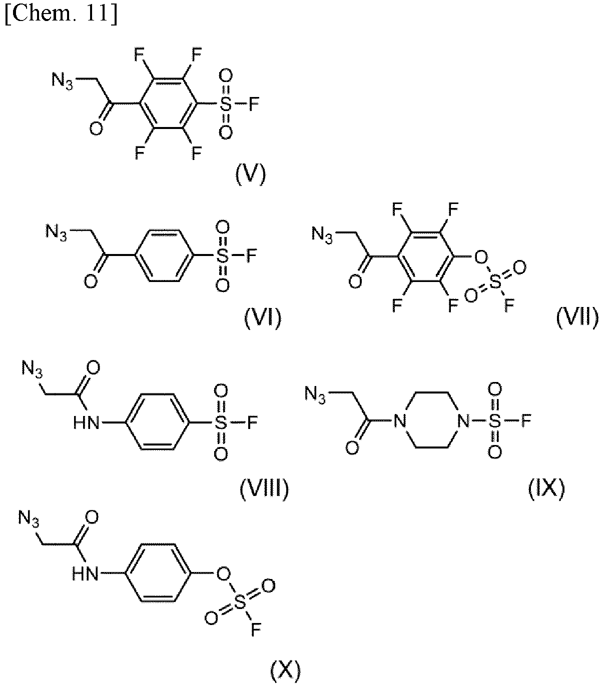

Accordingly, in an embodiment, the warhead compound has the following structure: 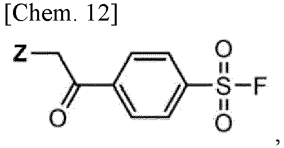 wherein Z may be an azide group or an alkynyl, cycloalkynyl, or heterocycloalkynyl group, or a different reactive group.

Specific reaction conditions for azide-alkyne click chemistry reactions are well known to those skilled in the art. For example, an azide-alkyne click chemistry reaction can be carried out by mixing an alkyne-structure-containing compound and an azide-group-containing compound in an aqueous solution in the presence of a copper(I)-stabilizing ligand such as Tris(3-hydroxypropyltriazolylmethyl)amine and a reducing agent such as ascorbic acid, and copper(II) sulfate. The copper catalysis system is not necessary for SPAAC. Azide-alkyne click chemistry reactions can be carried out at room temperature.

By an azide-alkyne click chemistry reaction, the above-described linking moiety derived from the alkyne structure and the azide group is formed. As a result, a structure in which the nucleic acid aptamer and the fluorosulfonyl group are linked via the linker comprising this linking moiety (which is called "third linker" in the present disclosure) is created. This structure can provide an embodiment of the neutralizable covalent drug compound as well as an active component of the neutralizable covalent drug. The third linker may be a divalent group having the structure: -(first linker)-(said linking moiety)-(second linker)-.

The present method may further comprise chemically synthesizing the nucleic acid aptamer of a) prior to performing the azide-alkyne click chemistry reaction. The nucleic acid aptamer components of a) in the present embodiments are, essentially, modified nucleotides, and as such can be synthesized by applying known oligonucleotide chemical synthesis methods. The phosphoramidite method is one example of a suitable chemical synthesis method, but examples are not necessarily limited to it.

For example, a nucleic acid aptamer linked or bound to the (a1) or (a2) group may be chemically synthesized, by chemically synthesizing an oligonucleotide using a nucleoside (*e.g*. in the form of a phosphoramidite derivative) having a nucleobase to which the (a1) or (a2) group is linked or bound, as the substrate for a desired residue in a desired sequence.

Nucleic acid aptamers can have high drug efficacy as well as safety due to their advantageous properties including the ability to provide high target-specificity, the antidotability, and the fact that they scarcely cause immune reactions by themselves, and therefore nucleic acid aptamers are considered promising pharmaceutical agents. On the other hand, the low stability *in vivo,* or more specifically the short half-life in the circulation due to removal in the kidney, is considered a shortcoming of the aptamer-based pharmaceutical agents and this is one reason why progress of their clinical applications is slow. The aptamer-based covalent drug provided by the embodiments of the present disclosure will bind to the target protein, and then once its warhead portion has formed a covalent bond against the target protein, the drug will be retained on or near the target protein, to be able to maintain the effect on the target protein. Therefore, the aptamer-based covalent drug can for example achieve a sustained drug effect in a pharmacokinetics different from that of a conventional aptamer-based drug having a passive and short half-life, and moreover, its drug effect can still be neutralized at desired timing.

### Examples

Below, examples will be shown to describe embodiments of the present disclosure in greater detail. However, these Examples are merely for illustrations, and the present invention is not limited to these specific embodiments illustrated. Materials and methods sections 3 to 5 only describe representative experimental conditions. The experimental conditions, particularly the reagent concentrations and the reaction times, were made consistent within a same set of experiments but may have been different between different sets of experiments.

### [Materials and Methods]

### 1. General

An unmodified thrombin-binding aptamer (TBA) consisting of the sequence 5'-GGT₃TGGTGT₉GGT₁₂TGG-3' (SEQ ID NO: 1) described in Bock et al., Nature, 1992, 355, 564-566, and three alkyne-linker-modified TBAs consisting of a sequence in which T at position 3, T at position 9, or T at position 12 in SEQ ID NO: 1 has been replaced with an alkyne-linker-modified T, respectively, were custom synthesized at Integrated DNA Technologies, Inc. These alkyne-linker-modified Ts have -C=C-(CH₂)₄-C≡CH, instead of the usual methyl group, bound to position 5 of the pyrimidine ring of the thymine (T) base. Here, -C≡C-(CH₂)₄- can be recognized as a first linker and -C=CH as an alkynyl group. Similarly, corresponding thiol-linker-modified TBAs were also custom synthesized. The thiol-linker-modified T has - CH=CH-CONH-(CH₂)₆-NHCO-(CH₂)₂-SH bound to position 5 of the pyrimidine ring of the thymine (T) base.

In a similar manner to the above, an unmodified SARS-CoV-2 spike protein binding aptamer consisting of the sequence 5'-CAGCACCGACCT₁₂TGTGCTTTGGGAGTGCT₂₉GGTCCAAGGGCGT₄₂TAATG GACA-3' (SEQ ID NO:2) described in Song et al., Anal. Chem., 2020, 92, 9895-9900, and three alkyne-linker-modified aptamers consisting of a sequence in which T at position 12, T at position 29, or T at position 42 in SEQ ID NO:2 has been replaced with an alkyne-linker-modified T, respectively, were synthesized. Further in a similar manner, an unmodified VEGF binding aptamer consisting of the sequence 5'-CAAT₄TGGGCCCGTCCGT₁₇ATGGT₂₂GGGT-3' (SEQ ID NO:3) described in Kaur and Yung, PLoS ONE, 2021, 7:e31196, and three alkyne-linker-modified aptamers consisting of a sequence in which T at position 4, T at position 17, or T at position 22 in SEQ ID NO:3 has been replaced with an alkyne-linker-modified T, respectively, were synthesized.

Human α thrombin was purchased from Haematologic Technologies, LLC, U.S.A. (#HCT-0020), fibrinogen from human plasma was purchased from Aldrich, U.S.A. (#9001-32-5), and human serum was purchased from Aldrich, U.S.A. (#H4522). The RBD of SARS-CoV-2 spike protein (#SPD-C52H3) was purchased from ACROBiosystems, U.S.A.

The synthesized warhead compound was identified with NMR, and linkage to the aptamers confirmed by HPLC. NMR experiments were performed by using a 500 MHz nuclear magnetic resonance device (JNM-ECA500, JEOL Ltd.) at 25°C. High performance liquid chromatography (HPLC) analyses were performed on an Agilent 1100 HPLC system (Agilent Technologies, Inc., U.S.A.) connected to a photodiode array (PDA) and/or LCQ-Fleet ion trap mass spectrometer and equipped with a C18 reversed-phase column (Hypersil GOLD, 2.1×100 mm, Thermo Fisher Scientific Inc., U.S.A.) using a 0-100% acetonitrile gradient comprising 0.1% formic acid at a 300 µL/min flow rate. A small scale quantitative analysis of the aptamers was carried out by using a reversed-phase semi-micro HPLC system (PU-2085 with C18 column, JASCO Corporation) connected to a fluorescence detector followed by a PDA. The aptamers were separated using a 0-60% acetonitrile gradient comprising a 20 mM triethylamine acetate aqueous solution (pH 7.4) for 26 minutes at a flow rate of 200 µL/min.

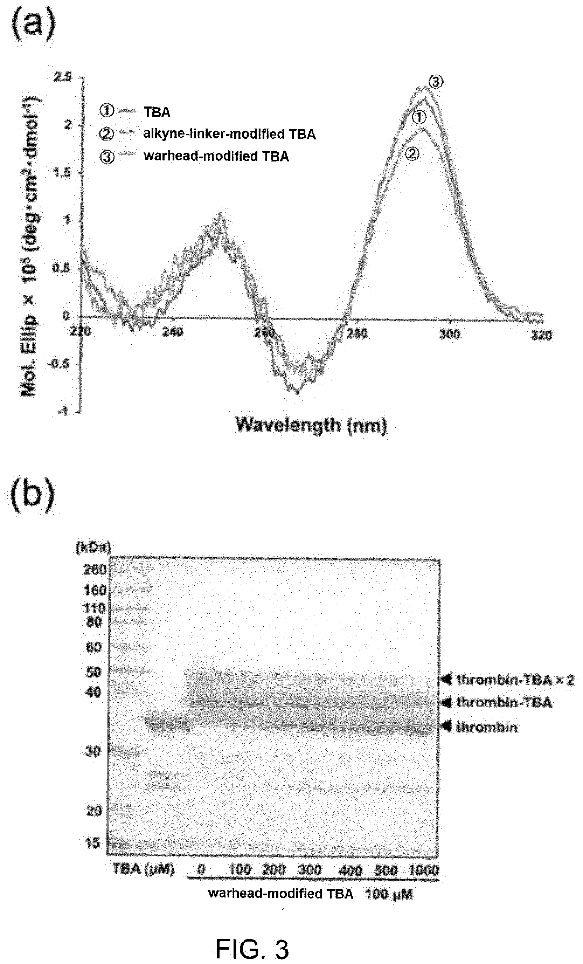

CD (circular dichroism) spectra of the unmodified TBA, alkyne-linker-modified TBA (T₃), and warhead-linked TBA (TBA₃) were measured. Specifically, 10 µM of the aptamers were held in D-PBS (pH 7.4) at 37°C and spectrum scanned between 220 and 340 nm at 100 nm/min (J-720W Circular Dichroism Spectrometer, Jasco Corporation). Triplicate measurements were averaged for the plot. The CD spectrum of the unmodified TBA was consistent with the previous report (Nagatoishi et al., Biochem. Biophys. Res. Commun., 2007, 352, 812-817) indicating that a G-quadruplex structure was present in the solution (Figure 3a).

All images of stained gels and in-gel fluorescence were captured by ChemDoc XRS+ (BIO-RAD Laboratories Inc., U.S.A.) and band intensities were quantified by using Image Lab^{™} software (BIO-RAD Laboratories Inc., U.S.A.).

### 2. Synthesis of covalent-binding aptamers

### 2.1. Synthesis of warhead compound 1

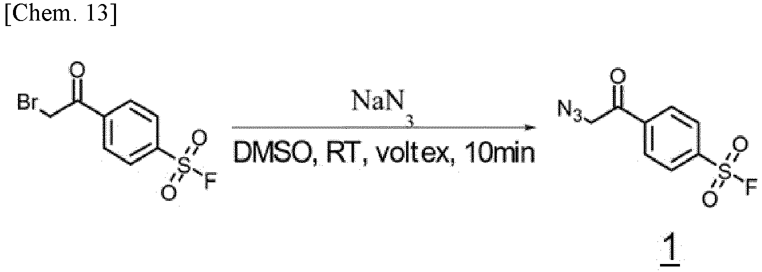

The warhead compound 1 was synthesized on a preparative scale according to the following procedure.

4-(2-bromoacetyl)-benzene-1-sulfonyl fluoride (71.1 µmol, Aldrich, U.S.A., #00364) and sodium azide (64.6 µmol, Wako Pure Chemical Industries, Ltd., #195-11092) were mixed in 323 µL of dimethyl sulfoxide (DMSO). The reaction mixture was vortexed for 10 minutes at room temperature, then mixed with cold water (0.5 mL) and extracted with ethyl acetate (1 mL). The collected organic phase was washed with saturated NaHCO₃ (0.5 mL ×2) and brine (0.5 mL ×2), dried over Na₂SO₄, and evaporated to afford a pure product as a yellow solid (11.8 mg, yield 49%). The warhead compound 1 shown on the right side of the figure above was identified by ¹H and ¹³C NMRs. In this compound, the acetyl-benzene moiety can be recognized as a second linker

### 2.2. Synthesis of aptamers linked to warhead

Tris(3-hydroxypropyltriazolylmethyl (0.5 µmol in water, Aldrich, U.S.A., #762342) and copper(II) sulfate (0.25 µmol in water, Aldrich, U.S.A., #451657) were mixed. Then, each alkyne-linker-modified aptamer (10 nmol in water) and 50 mM warhead compound 1 (0.5 µmol in DMSO) were added, and following further addition of ascorbic acid (0.4 µmol in water, Aldrich, U.S.A., #A92902), the mixture was reacted for 1 hour at room temperature. The reaction product was purified through ethanol precipitation. Specifically, the crude reaction product was combined with 3M sodium acetate (9 µmol in water) and ethanol, which had been cooled to - 20°C, and incubated at -20°C for 1hour. Then centrifugation was carried out (15000 rpm, 20 minutes, 4°C), supernatant was removed, and the precipitate was washed with a 70% ethanol solution and dissolved in nuclease-free water. The pure aptamers each linked to the warhead were identified by HPLC analysis.

For the thiol-linker-modified aptamers, the warhead compound 4-(2-bromoacetyl)-benzene-1-sulfonyl fluoride or ethenesulfonyl fluoride was reacted with the thiol group under the following condition to produce thioether linkage: A mixture of 50 µM thiol-linker-modified aptamer, 2.5 mM TCEP, and 10 mM warhead compound in 100 mM Tris-HCl pH 8.8 was reacted at 4°C for 3 hours, and then the warhead-modified aptamer was recovered by ethanol precipitation. If the former of the warhead compounds was used, linkage of a warhead in the form of carbonyl benzene-sulfonyl fluoride (BSF) was obtained, and if the latter of the warhead compounds was used, linkage of a warhead in the form of ethyl-sulfonyl fluoride (ESF) was obtained.

### 3. Covalent-bonding ability of aptamers linked to warheads

### 3.1. Covalent bond mobility-shift assay

Unmodified TBA, and TBAs to which the warhead was linked to any one of the three positions in the nucleic acid sequence (referred to as TBA₃, TBA₉, and TBA₁₂) (100 µM each) were each mixed with thrombin (25 µM) in phosphate-buffered saline (PBS, pH 7.4) and incubated at 37°C for 12 hours. The mixture was then combined with 1× sample buffer and separated by 12.5% sodium dodecyl sulfate poly-acrylamide gel electrophoresis (SDS-PAGE). Total proteins on the gel were visualized by Coomassie brilliant blue (CBB) staining. When TBA was covalently bonded to thrombin, it was detectable as a mobility shift of the thrombin on the gel. Although representative experiments pertaining to thrombin-binding aptamers have been described here, essentially the same experimental procedures were used for SARS-CoV-2 spike protein-binding aptamers and VEGF-binding aptamers.

To investigate concentration-dependency of TBA in the covalent bond formation, TBA₃ at different molar concentrations was mixed with thrombin at a constant concentration (25 µM) in PBS (pH7.4) and incubated at 37°C for 12 or 3 hours. Proteins were visualized by CBB staining of the SDS-PAGE gels, as described above. The bands of unreacted thrombin (*i.e.*, thrombin that had not undergone a covalent-bonding reaction with the aptamer) were quantified by Image Lab^{™} software. Intensity of the unreacted thrombin band in the sample with no TBA₃ addition was normalized to 100%, and relative intensity of that band in each of the other samples was determined. Competition experiments were also performed in which a constant concentration of TBA₃, a constant concentration of thrombin and different concentrations unmodified TBA were mixed.

### 3.2. Protein digestion and analysis of covalent bond sites by LC-MS/MS

TBA₃ (100 µM) and thrombin (25 µM) were mixed in PBS (pH 7.4) and incubated for 3 hours at 37°C. Proteins were visualized by CBB staining of the SDS-PAGE gels, as described above. The stained band corresponding to thrombin covalently bound by TBA was excised from the gel. Proteins in the gel were reduced with 25 mM dithiothreitol at 65°C for 10 minutes and then alkylated with iodoacetamide at room temperature for 1 hour in the dark. Digestion was carried out with modified trypsin (Promega Corporation, U.S.A., #V5111) in the presence of 25 mM n-octyl-beta-D-thioglucoside (Wako Pure Chemical Industries, Ltd., # 349-05361) for 12 hours at 37°C. The resulting peptides were analyzed by using LC-MS/PDA system. The trypsinized peptides were separated by using a 0%-50% acetonitrile gradient comprising 0.1% formic acid at a flow rate of 300 µL/min over 55 minutes and then eluted peptides were directly sprayed into the mass spectrometer. The mass spectrometer was operated in the data-dependent mode and externally calibrated. Survey MS scans were acquired in the 200-2000 m/z ranges. Multiply charged ions with high intensities per scan were fragmented with collision-induced dissociation in the ion trap. A dynamic exclusion window was applied within 30 seconds. All tandem mass spectra were collected by using normalized collision energy of 40 %. Data were acquired and analyzed with Xcalibur software v.2.07 (Thermo Scientific, U.S.A.).

### 4. Evaluation of thrombin-inhibiting activity

### 4.1. Evaluation of thrombin-inhibiting activity by turbidimetric assay

Different molar concentrations of unmodified TBA or TBA₃ and a constant molar concentration of thrombin (25 µM) were mixed in PBS (pH 7.4), and incubated for 3 hours at 37°C. Each reaction mixture was then added into a fibrinogen solution in PBS (pH 7.4) to give final concentrations of 2.5 nM thrombin and 1 mg/mL fibrinogen, and absorbance at the maximum absorbance wavelength for the polymerized fibrin (288 nm) was measured every 10 seconds by NanoPhotometer^{™} (Implen, Germany) using a 10-mm plastic cell. In each experiment, the absorbance at 0 second was normalized to 0, and the relative absorbance at each of the other time points was determined.

### 4.2. Evaluation of thrombin-inhibiting activity by changes in clotting time

Mixing was performed as described for 4.1. above, except that the final concentrations were 13 nM for thrombin and 2 mg/mL for fibrinogen. Changes in clotting time were measured by an automated coagulometer, BFT II Analyzer (SIEMENS Healthineers AG, Germany).

### 5. Confirmation of on-demand neutralization property

### 5.1. Binding of complementary strand antidote

The sequence of the complementary strand (*i.e*. antidote) DNA oligonucleotide for TBA was 5'-CCAACCACACCAACC-3' (SEQ ID NO:4). The same complementary strand labeled at 5'-end with fluorescein was also synthesized (referred to as FAM-modified complementary strand).

Thrombin (25 µM) was treated with or without TBA₃ (100 µM) in PBS (pH 7.4) and incubated for 3 hours at 37°C. For confirmation of binding specificity, an experiment was also performed in which this treatment was done in the presence of 40% (v/v) human serum. The mixtures were then treated with or without the FAM-labeled complementary strand (400 µM) for 30 minutes at 37°C. Each sample was mixed with 1× sample buffer, separated by 12.5% SDS-PAGE, and analyzed by in-gel fluorescence imaging. Total proteins were visualized by CBB staining.

### 5.2. Evaluation of neutralization of thrombin-inhibiting activity by turbidimetric assay

Unmodified TBA (3.5 nM) or TBA₃ (1.2 nM) were mixed with thrombin (25 µM) in PBS (pH 7.4) and incubated for 3 hours at 37°C. To each reaction mixture, a different concentration of the complementary strand antidote was then added, and it was incubated for 30 minutes at 37°C. Then, each reaction mixture was added into a fibrinogen solution in PBS (pH 7.4) to give final concentrations of 2.5 nM thrombin and 1 mg/mL fibrinogen, and absorbance at the maximum absorbance wavelength for the polymerized fibrin (288 nm) was measured every 10 seconds by NanoPhotometer^{™} using a 10-mm plastic cell. In each experiment, the absorbance at 0 second was normalized to 0, and the relative absorbance at each of the other time points was determined.

### 5.3. Evaluation of neutralization of thrombin-inhibiting activity by changes in clotting time

The same mixing was performed as in 5.2. above except the aptamers and the complementary strand antidote were added at the concentrations shown in Table 1 and the final concentrations were 13 nM thrombin and 2 mg/mL fibrinogen. Changes in clotting time were measured by BFT II Analyzer.

### 6. Statistical analysis

Values are presented as mean ± SD. Statistical significance of differences between groups was determined by a paired t-test. A p-value < 0.05 was considered statistically significant (∗p < 0.05, ∗∗p < 0.01, ∗∗∗p < 0.001, ∗∗∗∗p < 0.0001, *****p < 0.00001). The analyses were performed using GraphPad Prism 6.0 software (GraphPad Software Inc., U.S.A.).

### [Results and Discussion]

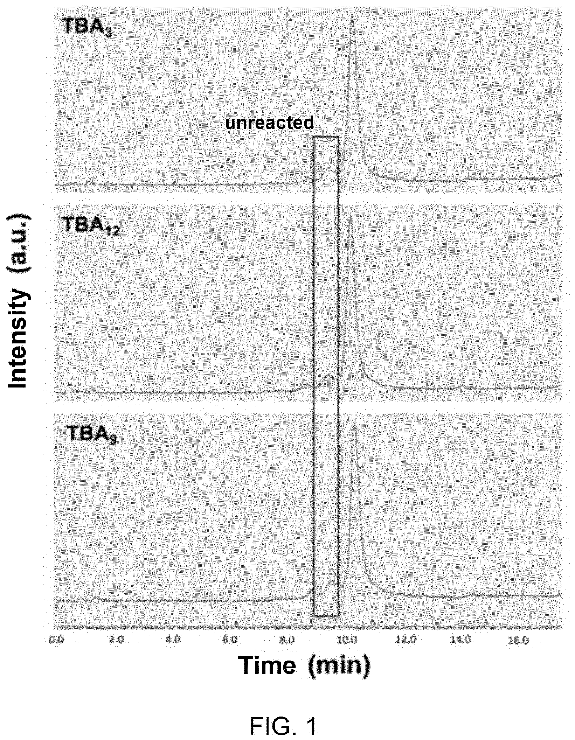

Figure 1 shows a result of an HPLC analysis of the reaction mixtures following the reactions to link the fluorosulfonyl group (warhead) to the nucleic acid aptamer. In Figure 1, the peaks corresponding to the unreacted materials, *i.e*. the aptamers which were linked to an alkynyl group but not linked to a fluorosulfonyl group, are boxed. The large peaks seen on the right side of the box correspond to the reaction products, *i.e*., the aptamers, which were further linked to a fluorosulfonyl group. It can be seen that warhead introduction efficiency of 90% or better was obtained, without any purification, at each site of the thrombin-binding aptamer (TBA) tested.

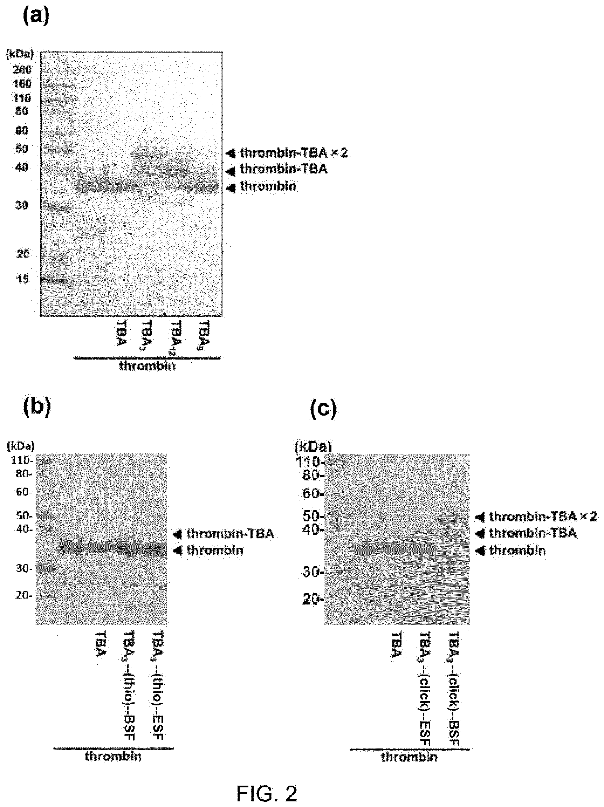

Figure 2 (a) shows a result of visualizing the proteins by CBB after incubating with thrombin the unmodified TBA or the TBAs linked to a fluorosulfonyl group via azide-alkyne click chemistry at 37°C for 12 hours, and then separating the reaction mixtures by SDS-PAGE. The 1^{st} lane of the gel shown contains molecular weight markers. Thrombin by itself has a molecular weight of about 36 kDa (2^{nd} lane). Even if this was bound by the unmodified TBA, mobility change of thrombin does not occur when run in an SDS-PAGE gel having a reducing/denaturing condition (3^{rd} lane). In contrast, when the TBA linked to a fluorosulfonyl group via a linker at position 3, position 12, or position 9 of the nucleic acid sequence (TBA₃, TBA₁₂, and TBA₉, respectively) was bound, the aptamer formed a covalent bond to thrombin, and thus a mobility shift of thrombin on SDS-PAGE, *i.e.* an increase in the molecular weight, was confirmed (4^{th}-6^{th} lanes, "thrombin-TBA"). For TBA₃, the band of the covalently-bound thrombin was excised from the gel, and mass spectrometry of the trypsinized peptide fragments was performed. As a result, the peptide IYIHPR was identified as at least one of the covalent-bonding sites (data not shown).

The results of Figure 2 (a) indicate that it was more or less possible to form a covalent bond even when the warhead attachment site was varied. The nucleic acid aptamer is not functioning as a genetic material but as a binding body, which as a whole provides great many interaction points. Although the warhead is linked to a thymine base in this particular experiment, it does not have to be thymine, and it can be appreciated that it is also possible to link the warhead to other bases, to the nucleic acid terminus, *etc.* An *in silico* simulation estimates that T₃ and T₁₂ of TBA are located nearly at the thrombin-binding face, whereas T₉ is removed from the thrombin-binding face. The observation that the covalent-bonding rate of T₉ (6^{th} lane) is lower than those of T₃ and T₁₂ is consistent with that estimate. Depending on individual applications, appropriate moderation of covalent-bonding rates may become beneficial. The present results indicate that the effect of the covalent drug may be modulated by changing the warhead linkage site.

In Figure 2 (a), the presence of a band corresponding to thrombin covalently bound by two aptamer molecules was also observed ("thrombin-TBA×2"). This result suggests that, during the 12 hour incubation period, the first aptamer molecule bound the thrombin molecule and formed a covalent bond, which was followed by temporary dissociation of the non-covalent binding of the aptamer portion, and then in that moment the second aptamer molecule bound the same thrombin molecule to form a covalent bond. An aptamer once dissociated, like this first aptamer molecule, may usually be washed away by the blood flow, but if it has formed a covalent bond it can be retained near the target to gain an opportunity for re-binding.

The right-most lane in Figure 2 (b) is a sample in which the same thiol linker as in Non-Patent Document 4 had been attached to position T₃ of TBA and the same alkyl-type ESF warhead as in Non-Patent Document 4 was thioether-linked ("thio") thereto. In striking contrast to the results of Figure 2 (a), the obtained warhead-modified aptamer was not at all able to form a covalent bond to the aptamer target. On the other hand, the second lane from the right in Figure 2 (c) is a sample in which this same ESF warhead was linked to position T₃ of TBA via azide-alkyne click chemistry ("click"). In this case, clear covalent bonding was obtained, albeit not to the level seen with the aryl-type BSF warhead (see Figure 2 (c), the right-most lane, and Figure 2 (a)). In a series of experiments performed by the inventors, significant superiority of the aryl-type warheads in the context of aptamer-based covalent drugs has been revealed. This can be recognized in the fact that there is a detectable covalent bonding in the second lane from the right in contrast to the right-most lane in Figure 2 (b), pertaining to thioether linkages, and in the comparison between the first and second lanes from the right in Figure 2 (c) which pertains to azide-alkyne click chemistry linkages. Taken together, the results of Figure 2 demonstrate importance of use of an azide-alkyne click chemistry linkage and/or use of an aryl-type warhead in the aptamer-based covalent drugs.

TBA₃ was used in the following experiments as it had had the highest covalent-bonding rates in the experiments of Figure 2. Also, in all the following Examples, a BSF warhead linked via azide-alkyne click chemistry was used. The structures of the alkyne-linker-modified TBA and the warhead-modified TBA were analyzed by CD spectra which revealed steric structures similar to that of an unmodified TBA (Figure 3a). Further, in an experiment in which the target thrombin and the warhead-modified TBA were mixed in the presence of an unmodified TBA, a clear competition between these TBAs was observed (Figure 3b) - the more the unmodified TBA was present, the less the warhead-modified TBA could form a covalent bond. Thus, it was revealed that the warhead-modified TBA and the unmodified TBA had similar binding sites on the target. The results of Figure 3 show that the linkage to the warhead had little or no impact on the basic properties inherent in the aptamer.

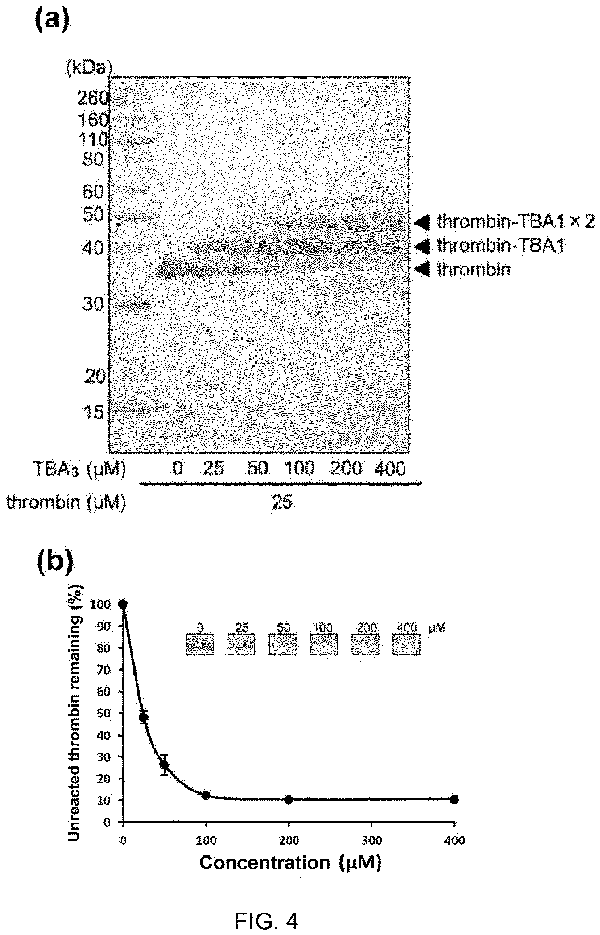

Figure 4 (a) provides data showing concentration dependency in the covalent-bonding reaction by the warhead-modified TBA. In Figure 4 (b), the intensity of the band of the unreacted thrombin which had not formed a covalent bond was quantified on the SDS-PAGE gel. At all concentrations tested, the warhead-modified TBA was able to recognize thrombin and quickly form a covalent bond at a human body temperature (37°C). It is believed that when the warhead-modified aptamer specifically but non-covalently binds the target protein, a nucleophilic amino acid on the target protein positioned near the fluorosulfonyl group reacts with the fluorosulfonyl group to form a covalent bond.

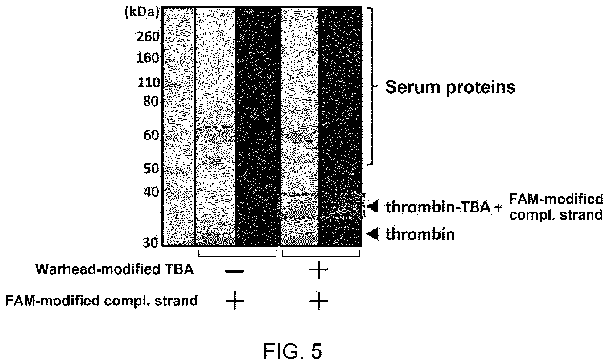

In the experiments of Figure 5, the warhead-modified TBA and thrombin were mixed in the presence of serum proteins. Then, the FAM-modified complementary strand was further added. The photographs of the in-gel fluorescence imaging detecting FAM-origin fluorescence are shown on the right sides of the photographs of CBB staining. Despite the presence of a large amount of serum proteins, it can be confirmed that only thrombin was covalently bound by the warhead-modified TBA to have a mobility shift, and that only the mobility-shifted thrombin molecule was bound to the FAM-modified complementary strand. These data demonstrate the high specificity of both the warhead-modified aptamer and the complementary strand.

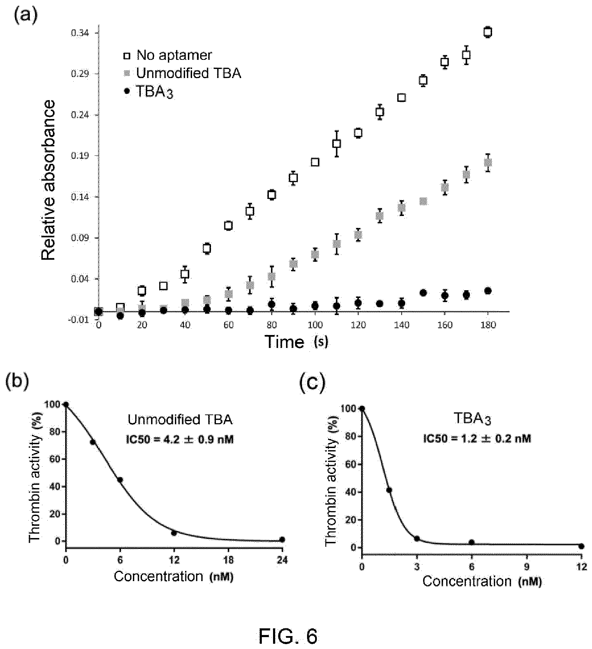

Thrombin is a serine protease enzyme which cleaves fibrinogen to produce fibrin, and this fibrin polymerizes to become polymerized fibrin. Polymerized fibrin can be quantified by measuring absorbance at 288 nm in a turbidimetric assay. In the experiments of Figure 6, inhibition of thrombin activity caused by TBA was investigated by looking at the activity to produce polymerized fibrin from fibrinogen. As seen in Figure 6 (a), TBA₃ had a significantly enhanced ability to inhibit thrombin compared to the unmodified TBA. Investigation of the aptamer concentration dependency revealed that the warhead-modified TBA₃ (Figure 6c) had about 3.5 times greater inhibition activity compared to the unmodified TBA (Figure 6b). That is, the warhead-modified TBA₃ could inhibit the thrombin activity at lower concentrations.

Further, an automated coagulometer was used to investigate changes of clotting time caused by thrombin inhibition. As a result, the warhead-modified TBA compared to the unmodified TBA prolonged the clotting time about 2 folds at the lower concentration (1 nM), and more than about 5 folds at the higher concentration (5 nM) (Table 1). The unmodified TBA could also prolong the clotting time to over 200 seconds if added at a high concentration of 100 nM (not shown in the Table).

**Table 1**

| Aptamer | Cone. | Compl. Strand | Clotting Time (s) | P value |
|---|---|---|---|---|
| None | - | - | 21 ± 2 | 0 |
| Unmodified TBA | 1 nM | - | 26 ± 4 | 0.0065*** |
| Warhead-modified TBA | 1 nM | - | 41 ± 7 | 0.00016**** |
| Unmodified TBA | 5 nM | - | 40 ± 7 | - |
| Warhead-modified TBA | 5 nM | - | > 200 | - |
| Warhead-modified TBA | 1 nM | 5 nM | 21 ± 1 | 0.80 |
| Warhead-modified TBA | 1 nM | 20 nM | 20 ± 1 | 0.86 |
| Warhead-modified TBA | 5 nM | 5 nM | 39 ± 1 | 0.0080*** |
| Warhead-modified TBA | 5 nM | 20 nM | 22 ± 2 | 0.19 |

| | | | | |
|---|---|---|---|---|
| ***P < 0.05, **P < 0.01, ***P** < **0.001, ****P < 0.0001** | | | | |

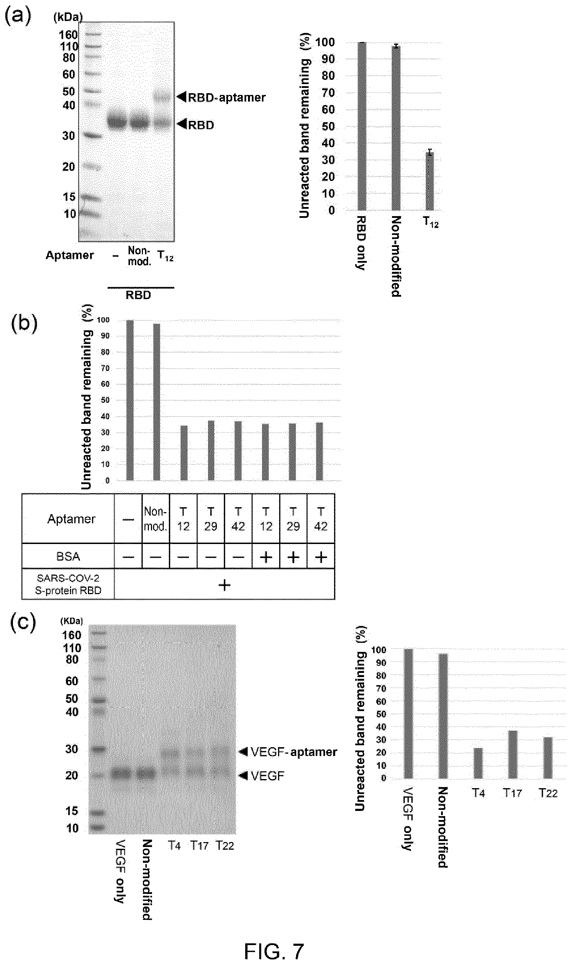

By using the similar technique, not only the thrombin-binding aptamer but any aptamers can be made covalent drugs. Figure 7 (a) shows a result of an SDS-PAGE mobility shift assay similar to the above; This was performed after incubating a warhead-modified aptamer, in which a fluorosulfonyl group was linked to the T nucleobase at position 12 of the SARS-CoV-2 spike protein-binding aptamer of SEQ ID NO:2, with the receptor binding domain (RBD) of the SARS-CoV-2 spike protein at 37°C for 12 hours. RBD is a protein domain responsible for viral binding to the human host cells, and said aptamer can inhibit the binding of RBD to the human receptor (Song *et al.*)*.* The warhead-modified aptamer caused a covalent binding reaction with RBD to generate a mobility shift on SDS-PAGE (Figure 7a, left). Figure 7a, right, shows the result of quantifying the proportion of the unreacted RBD band which had not formed a covalent bond (*i.e*., which did not shift). Similar results were obtained when the fluorosulfonyl group was linked to different positions within the aptamer (Figure 7b). Even when the incubation was done in the presence of nearly the same concentration of BSA (bovine serum albumin) as RBD, the covalent bonding of the aptamer to RBD was virtually unaffected (Figure 7b), nor was there a mobility shift of the BSA band (not shown in the figure). These data also demonstrate that the target specificity of the aptamer is not lost by the linkage to the warhead or by covalent bonding.

Figure 7 (c) shows a result of a similar mobility shift assay performed by mixing VEGF with the warhead-modified aptamers in which a fluorosulfonyl group was linked to different positions of the VEGF-binding aptamer. Each warhead-modified aptamer caused a covalent binding reaction with VEGF to generate a mobility shift on SDS-PAGE.

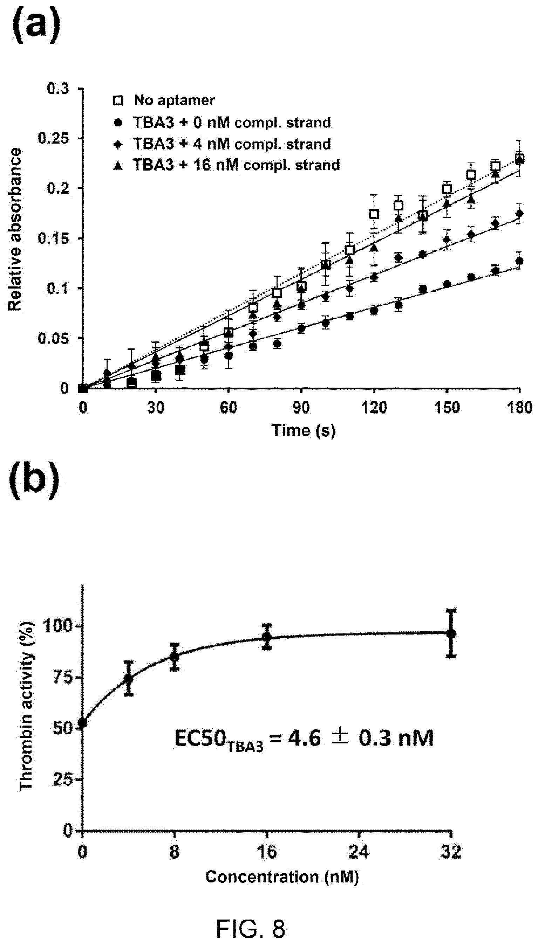

In addition to the high target specificity, the ability to be antidoted by a complementary strand is a characteristic of the aptamer-based drugs. The data of Figure 8 and the lower rows of Table 1 above confirm that this characteristic of the aptamer is retained even with the warhead linkage and covalent bonding. After 1.2 nM of the warhead-modified thrombin-binding aptamer (TBA₃) and thrombin were incubated at 37°C, different concentrations of the complementary strand were added and incubation was continued, and then fibrinogen was added, to measure thrombin activity based on the accumulation rate of polymerized fibrin (Figure 8) or the time needed for clotting (Table 1). The inhibition of thrombin by the warhead-modified TBA₃ was neutralized by the complementary strand in a concentration-dependent manner. When 16 nM of the complementary strand was added, thrombin activity was recovered to a state of almost no inhibition (Figure 8a). In this experimental system, EC₅₀ of the complementary strand was calculated to be 4.6±0.3 nM (Figure 8b). In a corresponding experiment using the unmodified TBA, EC₅₀ of 2.3±0.2 nM was obtained, suggesting that the unmodified TBA had a slightly higher sensitivity to the complementary strand (not shown in the figure), but it was clear from the results of Figure 8 that the effect of the covalently bound warhead-modified TBA could be neutralized by the complementary strand. The data in Table 1 relating to the clotting time also clearly supports the notion that the effect of the aptamer could be neutralized by administration of a complementary strand.

## Claims

1. A neutralizable covalent drug compound, comprising a nucleic acid aptamer; and a fluorosulfonyl group linked to the nucleic acid aptamer via an azide-alkyne click chemistry reaction,
wherein the aptamer and the fluorosulfonyl group are linked via a linker comprising a linking moiety formed by the azide-alkyne click chemistry reaction.

2. The neutralizable covalent drug compound according to claim 1,
wherein the linker is represented by a formula -L¹-Y-L²-, wherein Y is the linking moiety formed by the azide-alkyne click chemistry reaction, L¹ is a first linker moiety bound to the nucleic acid aptamer, and L² is a second linker moiety bound to the fluorosulfonyl group,
wherein within the second linker moiety, the end portion bound to the fluorosulfonyl group is an arylene.

3. The neutralizable covalent drug compound according to claim 2, wherein the arylene of the end portion bound to the fluorosulfonyl group is provided in the following structure:

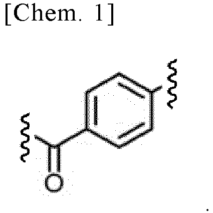

4. The neutralizable covalent drug compound according to any one of claims 1-3, wherein the linker is bound to a nucleobase in the nucleic acid aptamer.

5. The neutralizable covalent drug compound according to any one of claims 1-4, wherein the nucleic acid aptamer is a thrombin-binding aptamer having the sequence: 5'-GGTTGGTGTGGTTGG-3' (SEQ ID NO:1).

6. A pharmaceutical composition comprising the neutralizable covalent drug compound according to any one of claims 1-5.

7. An anticoagulant agent comprising the neutralizable covalent drug compound according to claim 5.

8. The anticoagulant agent according to claim 7 for use in a method of preventing or inhibiting blood coagulation in a patient, wherein the method comprises administering the anticoagulant agent to the patient and, following the administration of the anticoagulant agent, further administering an oligonucleotide complementary to the nucleic acid aptamer.

9. A neutralizable covalent drug system, comprising:
the compound according to any one of claims 1-5, the composition according to claim 6, or the anticoagulant agent according to claim 7 or 8; and
an oligonucleotide complementary to the nucleic acid aptamer.

10. A method of producing a neutralizable covalent drug capable of forming a covalent bond to a target protein, the method comprising reacting:
a) a nucleic acid aptamer specific to the target protein, wherein an alkynyl, cycloalkynyl, or heterocycloalkynyl group (a1) or an azide group (a2) is linked or bound to the aptamer; and
b) a warhead compound having a structure in which a corresponding azide group (b1) or alkynyl, cycloalkynyl, or heterocycloalkynyl group (b2) is linked or bound to a fluorosulfonyl group
to carry out an azide-alkyne click chemistry reaction, and
obtaining a structure in which the nucleic acid aptamer and the fluorosulfonyl group are linked via a linker comprising a linking moiety formed by the azide-alkyne click chemistry reaction.

11. The method according to claim 10, wherein the warhead compound comprises a second linker linking the fluorosulfonyl group to the azide group or the alkynyl, cycloalkynyl, or heterocycloalkynyl group, wherein within the second linker, the end portion bound to the fluorosulfonyl group is an arylene.

12. The method according to claim 11, wherein the arylene of the end portion bound to the fluorosulfonyl group is provided in the following structure:

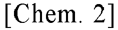

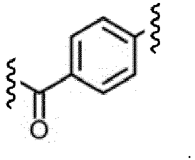

13. A neutralizable covalent drug compound, comprising a nucleic acid aptamer; and a fluorosulfonyl group linked to the nucleic acid aptamer via a linker, wherein within the linker, the end portion bound to the fluorosulfonyl group is an arylene.

14. The neutralizable covalent drug compound according to claim 13, wherein the arylene of the end portion bound to the fluorosulfonyl group is provided in the following structure:
